Europäisches Patentamt

European Patent Office

Office européen des brevets

(19)

(11) Publication number: **0 144 914**
A2

# (12) EUROPEAN PATENT APPLICATION

(21) Application number: 84114444.7

(51) Int. Cl.⁴: **C 12 Q 1/68**

(22) Date of filing: 29.11.84

(30) Priority: 12.12.83 US 560429
31.08.84 US 645850
07.11.84 US 668257

(43) Date of publication of application:
19.06.85 Bulletin 85/25

(84) Designated Contracting States:
AT BE CH DE FR GB IT LI LU NL SE

(71) Applicant: MILES LABORATORIES, INC.
1127 Myrtle Street
Elkhart Indiana 46514(US)

(72) Inventor: Albarella, James P.
25845 Meadow Oak Lane
Elkhart Indiana 46514(US)

(72) Inventor: Anderson DeRiemer, Leslie H.
634 Hollyridge Dr.
Encinitas CA 92024(US)

(72) Inventor: Carrico, Robert J.
54256 Silver Street
Elkhart Indiana 46514(US)

(74) Representative: Adrian, Albert, Dr. et al,
c/o BAYER AG Konzernverwaltung RP Patentabteilung
D-5090 Leverkusen 1, Bayerwerk(DE)

(54) Hybridization assay employing labeled pairs of hybrid binding reagents.

(57) A nucleic acid hybridization assay wherein the hybrid formed with the probe has binding sites for two specific binding reagents, one of which comprises a first label and the other a second label, interaction of the first and second labels providing a detectable response which is measurably different when the two labeled reagents are both bound to the same hybrid compared to when the two labeled reagents are not so bound. The formation of the hybrid assay product brings the two labels within a proximate interaction distance of one another, e.g., as in the cases of sequential catalyst (enzyme) interaction and energy transfer. Since the labels provide a response which is distinguishable when they are associated with a hybridized probe, no separation step is required.

Croydon Printing Company Ltd.

## HYBRIDIZATION ASSAY EMPLOYING
## LABELED PAIRS OF HYBRID BINDING REAGENTS

### FIELD OF THE INVENTION

This invention relates to nucleic acid hybridization assay methods and reagent systems for detecting specific polynucleotide sequences. The principle of nucleic acid hybridization assays was developed by workers in the recombinant DNA field as a means for determining and isolating particular polynucleotide base sequences of interest. It was found that single stranded nucleic acids, e.g., DNA and RNA, such as obtained by denaturing their double stranded forms, will hybridize or recombine under appropriate conditions with complementary single stranded nucleic acids. By labeling such complementary probe nucleic acids with some readily detectable chemical group, it was then made possible to detect the presence of any polynucleotide sequence of interest in a test medium containing sample nucleic acids in single stranded form.

In addition to the recombinant DNA field, the analytical hybridization technique can be applied to the detection of polynucleotides of importance in the fields of human and veterinary medicine, agriculture, and food science, among others. In particular, the technique can be used to detect and

MS-1353

identify etiological agents such as bacteria and viruses, to screen bacteria for antibiotic resistance, to aid in the diagnosis of genetic disorders such as sickle cell anemia and thalassemia, and to detect cancerous cells. A general review of the technique and its present and future significance is provided in Biotechnology (August 1983), pp. 471-478.

## INFORMATION DISCLOSURE

The following information is provided for the purpose of making known information believed by the applicant to be of possible relevance to the present invention. No admission is necessarily intended, nor should be construed, that any of the following information constitutes prior art against the present invention.

The state-of-the-art nucleic acid hybridization assay techniques generally involve a separation of hybridized and unhybridized labeled probe. This required separation step is usually facilitated by immobilizing either the sample nucleic acids or the nucleic acid probe on a solid support. Commonly, hybridization between particular base sequences or genes of interest in the sample nucleic acids and a labeled form of the probe nucleic acid is detected by separating the solid support from the remaining reaction mixture which contains unhybridized probe, followed by detection of the label on the solid support.

There are continuing efforts to simplify the analytical procedure for performing nucleic acid

MS-1353

hybridization assays. A primary goal of these efforts is to reduce the complexity of the procedure to the point that it can be conveniently and routinely performed in clinical laboratories. The necessity of a separation step seriously impedes the progress of these efforts. The separation step requires considerable expertise in order to be accomplished in an analytically reproducible manner and is a physical manipulation not readily automated or suited to high volume testing.

Moreover, in conventional methods involving the immobilization of sample nucleic acids, two significant difficulties are encountered. Firstly, the procedures required to accomplish immobilization are generally time consuming and add a further step which is undesirable for routine use of the technique in a clinical laboratory. Secondly, proteins and other materials in the heterogeneous sample, particularly in the case of clinical samples, can interfere with the immobilization process.

As alternatives to immobilizing sample nucleic acids and adding labeled probe, one can use an immobilized probe and label the sample nucleic acids *in situ*, or one can use a dual hybridization technique requiring two probes, one of which is immobilized and the other labeled. The former alternative, however, is even less desirable since the *in situ* labeling of the sample nucleic acids requires a high degree of technical skill which is not routinely found in clinical technicians and there are no simple, reliable methods for monitoring the labeling yield, which can be a significant problem if the labeling media contain variable amounts of inhibitors of the labeling reaction. The dual hybridization technique has the disadvantages of requiring an additional reagent and incubation step and the kinetics of the hybridization reaction can be slow and inefficient. The accuracy of the assay can also be variable if the complementarity of the two probes with the sample sequence is variable.

Some of the problems discussed above are solved by employing an immobilized RNA probe and detecting resulting immobilized DNA·RNA or RNA·RNA hybrids with a labeled specific anti-hybrid antibody [see commonly assigned U.S. Patent Application Serial No. 616,132, filed June 1, 1984]. This technique still requires a separation step and thus has the disadvantages common to all hybridization techniques that require a separation step as discussed above.

U.S. Patent Nos. 3,996,345; 4,233,402; and 4,275,149 describe assays to detect antigens by immunoassay techniques involving enzyme pair and energy transfer interactions.

European Patent Application No. 70,685 proposes a hybridization assay technique that dispenses with the need to physically separate hybridized from unhybridized probe. It is proposed to employ a pair of probes which hybridize to contiguous regions on a polynucleotide sequence of interest and to label one probe with a chemiluminescent catalyst such as the enzyme peroxidase and the other with an absorber molecule for the chemiluminescent emission. The catalyst and absorber labels must be situated near the contiguous terminal ends of the respective probes such that upon hybridization there is observed quenching of the chemiluminescent emission by energy tranfer to the absorber molecule. In order to perform such an assay, one must be able to controllably synthesize two critical probe reagents such that the respective labels are brought into a quenching orientation upon hybridization to the sample nucleic acid and without affecting the affinity of the respectively labeled probe segments to actually undergo hybridization.

## SUMMARY OF THE INVENTION

A nucleic acid hybridization assay has now been devised based on the use of proximal interacting labeled pairs which yield a detectable signal that is measurably different for the hybridized probe compared to unhybridized probe. In this way there is no need to separate hybridized and unhybridized probe, greatly facilitating the performance and automation of the assay. In addition, the assay signal is nonradioisotopic in nature thereby meeting another criterion of assay convenience, the use of detection systems not involving radioactivity.

According to the present invention, specific polynucleotide sequences are detected in a test sample by forming a hybrid between the sequence to be detected and a nucleic acid probe having a complementary sequence such that the resulting hybrid possesses binding sites for two different specific binding reagents. One of such binding reagents comprises a first label and the other a second label where interaction between the two labels provides a detectable response which is measurably different, either in a positive or a negative sense, when the two labeled reagents are bound to the same hybrid compared to when not so bound. When bound to the same hybrid the two labels are brought to within a proximate interaction distance of one another, thereby substantially increasing signal affecting interactions between the two labels compared to the relatively less frequent interactions occurring in the bulk solution between the free diffusible labeled reagents.

A preferred interaction between the two labels is a sequential interaction wherein the first label participates in a first chemical reaction to produce a diffusible product that is a participant in a second chemical reaction with the second label to produce a detectable product. It is especially preferred that the first and second labels be catalysts, e.g., enzymes, for the first and second chemical reactions, respectively. For example, where the first label is glucose oxidase and the second is peroxidase, hydrogen peroxide produced by action of glucose oxidase on glucose diffuses to the peroxidase label to be detectable as an optical signal in the presence of suitable indicator dye compositions. Another preferred labeling pair is that involving energy transfer interaction such as between a fluorescer or luminescer and a quencher for the photoemission of the first label.

With regard to the nature of the specific binding agents involved, in principle at least one of the two must bind to a site which is unique to

MS-1353

the hybrid formed in the assay compared to unhybridized nucleic acids since signal generation must be dependent upon the formation of such hybrid. Preferably then, such specific binding reagent will be an antibody selective for the hybrid over the single stranded nucleic acids present in the assay mixture. A variety of second binding agents can be used as is detailed below.

The present invention is characterized by a number of significant advantages. In addition to the principal advantage of elimination of the separation step, there is no requirement to immobilize either sample or probe nucleic acids which gives rise to nonspecific binding and reproducibility problems in commonly used hybridization techniques. Further, the hybridization kinetics are substantially faster in solution compared to systems with one strand of the hybridizable pair immobilized. An additional advantage is that the assay can be performed without wash steps. The assay reagents can be sequentially added to the hybridization medium without the need to wash insoluble support materials.

Another significant feature of the present invention is that the detection systems involved can be particularly efficient since the double stranded duplexes can contain many binding sites for the first and second labeled reagents. This results in large amounts of the first and second labels becoming assembled into their interactive configuration per unit of hybridized probe. In considering antibodies to RNA·DNA or RNA·RNA hybrids, one labeled antibody can bind for approximately each 10 base pairs of the hybrid. If the probe is for example 500 bases long, 40-50 antibodies could bind. Ligands recognized by labeled binding proteins can be introduced into probes at levels of one ligand per 5-20 bases. The presence of multiple binding sites on hybrids can be used advantageously when low levels of hybrid must be detected.

BRIEF DESCRIPTION OF THE DRAWINGS

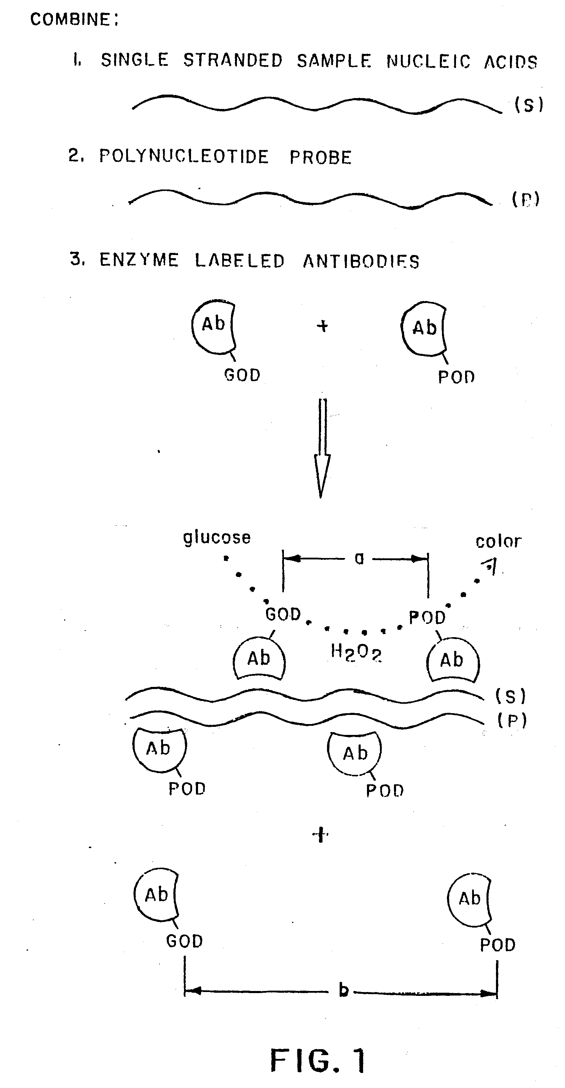

Figs. 1-3 are schematic illustrations of preferred methods for performing the present invention. These methods are described in detail below.

DESCRIPTION OF THE PREFERRED EMBODIMENTS

The use of nucleic acid hybridization as an analytical tool is based fundamentally on the double-stranded, duplex structure of DNA. The hydrogen bonds between the purine and pyrimidine bases of the respective strands in double-stranded DNA can be reversibly broken. The two complementary single strands of DNA resulting from this "melting" or "denaturation" of DNA will associate (sometimes referred to as reannealing or hybridization) to reform the duplexed structure. As is now well known in the art, contact of a first single stranded nucleic acid, either DNA or RNA, which comprises a base sequence sufficiently complementary to (i.e., "homologous with") a second single stranded nucleic acid under appropriate conditions, will result in the formation of

MS-1353

DNA·DNA, RNA·DNA, or RNA·RNA hybrids, as the case may be.

## The Probe

The probe will comprise at least one single stranded base sequence substantially complementary to or homologous with the sequence to be detected. However, such base sequence need not be a single continuous polynucleotide segment, but can be comprised of two or more individual segments interrupted by nonhomologous sequences. These nonhomologous sequences can be linear, or they can be self-complementary and form hairpin loops. In addition, the homologous region of the probe can be flanked at the 3'- and 5'-termini by nonhomologous sequences, such as those comprising the DNA or RNA of a vector into which the homologous sequence had been inserted for propagation. In either instance, the probe as presented as an analytical reagent will exhibit detectable hybridization at one or more points with sample nucleic acids of interest. Linear or circular single stranded polynucleotides can be used as the probe element, with major or minor portions being duplexed with a complementary polynucleotide strand or strands, provided that the critical homologous segment or segments are in single stranded form and available for hybridization with sample DNA or RNA. It will generally be preferred to employ probes which are substantially in single stranded form. The preparation of a suitable probe for a particular assay is a matter of routine skill in the art.

MS-1353

*Labeling Pairs*

According to the principle of the present invention, hybridization dependent upon the presence of the polynucleotide sequence of interest and binding of the labeled reagents to the hybrid results in the bringing together of the two labels within a certain distance such that the signal produced by their interaction is measurably different from that produced by their encounters during simple diffusion in the bulk assay medium. Various interaction phenomena can be applied to the present invention. The interaction can be chemical, physical, or electrical, or combinations of these forces. The environment of the labels which is created upon hybridization and binding of the labeled reagents to the formed hybrid, herein referred to as the bound hybrid environment, must be distinctively different in at least one critical aspect from the bulk medium. Since hybridization determines the proportion of labels which result in the localized hybrid environment compared to the bulk phase, the resulting signal response is dependent upon the presence of the sequence to be determined in the assay medium.

A preferred interaction between the two labels involves two chemical reactions wherein one label participates in the first reaction to produce a diffusible mediator product which participates in the second reaction with the second label to yield a detectable product. The microenvironment of the bound hybrid will thus contain a higher localized concentration of the mediator product, so as to increase the rate of the signal producing second

MS-1353

label reaction, than the bulk solution. The two labels, respectively, can participate in the reactions as reactants or, as is particularly preferred, catalysts. Any involved catalysis can be either enzymatic or nonenzymatic.

A variety of enzymes and catalysts can be applied to the present invention and their selection will be a matter of choice to one working in the field. Useful enzymes for the first reaction include the oxidoreductases, particularly those involving nucleotides such as nicotinamide adenine dinucleotide (NAD) or its reduced form (NADH), or adenosine triphosphate (ATP) as cofactors or those producing hydrogen peroxide or other small diffusible products. A few examples are alcohol dehydrogenase, glycerol dehydrogenase, lactate dehydrogenase, malate dehydrogenase, glucose-6-phosphate dehydrogenase, glucose oxidase, and uricase. Other classes of enzymes such as hydrolases, transferases, lyases, and isomerases can also be employed. It is particularly preferred that the second label also be an enzyme where a product of the first enzyme reaction is a substrate or cofactor for the second enzyme so as to result in rapid enzyme reaction in the environment of the bound hybrid. For example, where the first enzyme is an oxidoreductase producing hydrogen peroxide as a product, the second enzyme can be peroxidase. A detailed listing and description of useful enzymes is provided in U.S. Patent Nos. 4,233,402 and 4,275,149 incorporated herein by reference. Other useful systems involve one enzyme as the first label which catalyses a reaction producing a prosthetic group for an apoenzyme or proenzyme.

MS-1353

Nonenzymatic catalysts can also serve as labels as previously mentioned. By way of example, reference is made to U.S. Patent No. 4,160,645.

Another preferred interaction between the labels is that of energy transfer. The first label will be a photoemitting substance such as a fluorescer or luminescer, the former producing an emission upon irradiation and the second producing an emission upon chemical reaction. The photoemission is absorbable by the second label to either quench the emission or to provide a second emission such as where the absorbing label is a fluorescer itself. Pairings of compounds useful for this effect are described in detail in U.S. Patent Nos. 4,275,149 and 4,318,981. Some preferred fluorescer/quencher pairs are naphthalene/anthracene, α-naphthylamine/dansyl, tryptophan/dansyl, dansyl/fluorescein, fluorescein/rhodamine, tryptophan/fluorescein, N-[p-(2-benzoxazolyl)phenyl]maleimide (BPM)/thiochrome, BPM/8-anilino-1-naphthalenesulfonate (ANS), thiochrome/N-(4-dimethylamino-3,5-dinitrophenyl) maleimide (DDPM), and ANS/DDPM. Some preferred luminescer/quencher pairs are luminol with fluorescein, eosin or rhodamine S.

If desired, various modifications of the labeled reagents can be employed without departing from the scope of the present invention. In one variation, one of the labeled reagents comprises a solid phase to which is linked the binding substance and separately the label. The solid phase can be the walls of the reaction container or a dispersed solid such as a polyacrylamide or agarose bead. One could also modify the binding substance with a bindable ligand such as a hapten or biotin and introduce the label by addition of a labeled anti-hapten antibody or labeled avidin before or after the hybridization reaction. Thus, it can be seen that the label can be linked to the binding substance directly or indirectly through intermediary components.

## *The Binding Reagents*

A critical aspect of the present invention is the formation of a hybrid between the probe and the polynucleotide sequence of interest which comprises binding sites for the two specific binding reagents. A principle of the assay is that hybridization of the probe with the desired sequence results in a bringing together of the respective binding sites for the two binding reagents such that the labels are brought within a proximate interaction distance of one another. Any design of the system can be used which results in a binding site for at least one of the two labeled reagents which is unique to the hybrid. It will thereby be assured that the desired localization of the labeling pair will occur only upon formation of the hybrid.

The binding of the labeled reagents to the hybrid will normally involve a highly specific noncovalent binding such as is characteric of a variety of biologically derived substances, particularly binding proteins such as immunoglobulins. A variety of binding substances can be used to provide at least one binding reagent which has a unique binding affinity for the hybrid with inconsequential binding affinity for single stranded nucleic acids such as unhybridized probe and unhybridized sample nucleic acids.

Particularly preferred binding substances are antibody reagents having anti-hybrid binding activity and can be whole antibodies or fragments thereof, or aggregates or conjugates thereof, of the conventional polyclonal or monoclonal variety. Preferred antibody reagents will be those that are selective for binding (i) DNA·RNA or RNA·RNA hybrids or (ii) intercalation complexes. It is currently known that antibodies can be stimulated which are selective for DNA·RNA or RNA·RNA hybrids over the single stranded nucleic acids, however, it is presently considered infeasible to generate such selectivity in the case of DNA·DNA hybrids. To the extent that selective DNA·DNA antibodies are developed in the future, they will clearly be applicable to the present invention. Antibodies to DNA·RNA or RNA·RNA hybrids can be used where the probe is RNA and the sample nucleic acids are DNA or RNA or where the probe is DNA and the sample RNA.

Further, it should be understood that in referring to an RNA probe used with an anti-DNA·RNA or anti-RNA·RNA reagent, it is contemplated herein that not all nucleotides comprised in the probe be ribonucleotides, i.e., bearing a 2'-hydroxyl group. The fundamental feature of an RNA probe as used herein is that it be sufficiently non-DNA in character to enable the stimulation of antibodies to DNA·RNA or RNA·RNA hybrids comprising an RNA probe which do not crossreact to an analytically significant degree with the individual single strands forming such hybrids. Therefore, one or more of the 2'-positions on the nucleotides comprised in the probe can be in the deoxy form provided the antibody binding characteristics necessary for performance of the present assay are maintained to a substantial degree. Likewise, in addition or alternatively to such limited 2'-deoxy modification, an RNA probe can comprise nucleotides having other 2'-modifications, or in general any other modification along its ribose phosphate backbone provided there is not substantial interference with the specificity of the antibody to the double stranded hybridization product compared to its individual single strands.

Where such modifications exist in an RNA probe, the immunogen used to raise the antibody reagent would preferably comprise one strand having substantially corresponding modifications and the other strand being substantially unmodified RNA or DNA, depending on whether sample RNA or DNA is intended to be detected. Preferably, the modified strand in the immunogen would be identical to the modified strand in an RNA probe. An example of an immunogen is the hybrid poly(2'-0-methyladenylic acid)·poly(2'-deoxythymidylic acid). Another would be poly(2'-0-ethylinosinic acid)·poly(ribocytidylic acid). The following are further examples of modified nucleotides which could be comprised in an RNA probe: 2'-0-methylribonucleotide, 2-0 -ethylribonucleotide, 2'-azidodeoxyribonucleotide, 2'-chlorodeoxyribonucleotide, 2'-0 -acetylribonucleotide, and the methyl phosphonates or phosphorothiolates of ribonucleotides or

MS-1353

deoxyribonucleotides. Modified nucleotides can appear in RNA probes as a result of introduction during enzymic synthesis of the probe from a template. For example, adenosine 5'-0-(l-thiotriphosphate) (ATPαS) and dATPαS are substrates for DNA dependent RNA polymerases and DNA polymerases, respectively. Alternatively, the chemical modification can be introduced after the probe has been prepared. For example, an RNA probe can be 2'-0-acetylated with acetic anhydride under mild conditions in an aqueous solvent.

Immunogens for stimulating antibodies specific for RNA·DNA hybrids can comprise homopolymeric or heteropolymeric polynucleotide duplexes. Among the possible homopolymer duplexes, particularly preferred is poly(rA)·poly(dT) [Kitagawa and Stollar (1982) Mol. Immunol. 19:413]. However, in general, heteropolymer duplexes will be preferably used and can be prepared in a variety of ways, including transcription of φX174 virion DNA with RNA polymerase [Nakazato (1980) Biochem. 19:2835]. The selected RNA·DNA duplexes are adsorbed to a methylated protein, or otherwise linked to a conventional immunogenic carrier material, such as bovine serum albumin, and injected into the desired host animal [see also Stollar (1980) Meth. Enzymol. 70:70]. Antibodies to RNA·RNA duplexes can be raised against double stranded RNAs from viruses such as reovirus or Fiji disease virus which infects sugar cane, among others. Also, homopolymer duplexes such as poly(rI)·poly(rC) or poly(rA)·poly(rU), among others, can be used for immunization as above. Further information regarding antibodies to RNA·DNA and RNA·RNA hybrids is provided in commonly assigned U.S. Patent Application Serial No. 616,132, filed June 1, 1984.

Antibodies to intercalation complexes can be prepared against an immunogen which will usually comprise an ionic complex between a cationic protein or protein derivative (e.g., methylated bovine serum albumin) and the anionic intercalator-nucleic acid complex. Ideally, the intercalator will be covalently coupled to the double stranded nucleic acid. The intercalator-nucleic acid conjugate alternatively can be covalently coupled to a carrier protein. The nucleic acid portion of the immunogen can comprise the specific paired sequences found in the assay hybrid or can comprise any other desirable sequences since the specificity of the antibody will generally not be dependent upon the particular base sequences involved. Further information regarding antibodies to intercalation complexes is provided in commonly assigned U.S. Patent Application Serial No. 560,429, filed December 12, 1983.

As stated above, the antibody reagent can consist of whole antibodies, antibody fragments, polyfunctional antibody aggregates, or in general any substance comprising one or more specific binding sites from an antibody. When in the form of whole antibody, it can belong to any of the classes and subclasses of known immunoglobulins, e.g., IgG, IgM, and so forth. Any fragment of any such antibody which retains specific binding affinity for the hybridized probe can also be employed, for instance, the fragments of IgG conventionally known as Fab, F(ab'), and F(ab')$_2$.

MS-1353

In addition, aggregates, polymers, derivatives and conjugates of immunoglobulins or their fragments can be used where appropriate.

The immunoglobulin source for the antibody reagent can be obtained in any available manner such as conventional antiserum and monoclonal techniques. Antiserum can be obtained by well-established techniques involving immunization of an animal, such as a mouse, rabbit, guinea pig or goat, with an appropriate immunogen. The immunoglobulins can also be obtained by somatic cell hybridization techniques, such resulting in what are commonly referred to as monoclonal antibodies, also involving the use of an appropriate immunogen.

In those instances where an antibody reagent selective for intercalation complexes is employed as one of the binding reagents, a variety of intercalator compounds can be involved. In general it can be said that the intercalator compound preferably is a low molecular weight, planar, usually aromatic but sometimes polycyclic, molecule capable of binding with double stranded nucleic acids, e.g., DNA·DNA, DNA·RNA, or RNA·RNA duplexes, usually by insertion between base pairs. The primary binding mechanism will usually be noncovalent, with covalent binding occurring as a second step where the intercalator has reactive or activatable chemical groups which will form covalent bonds with neighboring chemical groups on one or both of the intercalated duplex strands. The result of intercalation is the spreading of adjacent base pairs to about twice their normal separation distance, leading to an increase in

MS-1353

molecular length of the duplex. Further, unwinding of the double helix of about 12 to 36 degrees must occur in order to accomodate the intercalator. General reviews and further information can be obtained from Lerman, J. Mol. Biol. 3:18(1961); Bloomfield et al, "Physical Chemistry of Nucleic Acids", Chapter 7, pp. 429-476, Harper and Rowe, NY(1974); Waring, Nature 219:1320 (1968); Hartmann et al, Angew. Chem., Engl. Ed. 7:693(1968); Lippard, Accts. Chem. Res. 11:211(1978); Wilson, Intercalation Chemistry(1982),445; and Berman et al, Ann. Rev. Biophys. Bioeng. 10:87(1981); as well as from the above-referenced U.S. Serial No. 560,429. Exemplary of intercalators are acridine dyes, e.g., acridine orange, the phenanthridines, e.g., ethidium, the phenazines, furocoumarins, phenothiazines, and quinolines.

The intercalation complexes are formed in the assay medium during hybridization by use of a probe which has been modified in its complementary, single stranded region to have the intercalator chemically linked thereto such that upon hybridization the intercalation complexes are formed. Essentially any convenient method can be used to accomplish such linkage. Usually, the linkage is formed by effecting intercalation with a reactive, preferably photoreactive intercalator, followed by the linking reaction. A particularly useful method involves the azidointercalators. Upon exposure to long wavelength ultraviolet or visible light, the reactive nitrenes are readily generated. The nitrenes of arylazides prefer insertion reactions over their rearrangement products [see White et al, Methods in Enzymol.

MS-1353

46:644(1977)]. Representative azidointercalators are 3-azidoacridine, 9-azidoacridine, ethidium monoazides, ethidium diazide, ethidium dimer azide [Mitchell et al, JACS 104:4265(1982)], 4-azido-7-chloroquinoline, and 2-azidofluorene. Other useful photoreactable intercalators are the furocoumarins which form [2+2] cycloadducts with pyrimidine residues. Alkylating agents can also be used such as bis-chloroethylamines and epoxides or aziridines, e.g., aflatoxins, polycyclic hydrocarbon epoxides, mitomycin, and norphillin A. The intercalator-modified duplex is then denatured to yield the modified single stranded probe.

Repeating what is stated previously, at least one of the binding reagents must be of the type discussed above, however, there is a greater latitude of choice possible for the other binding reagent since it need not discriminate between the hybrid and other nucleic acids, including the probe and sample materials, to accomplish the desired effect. Accordingly, the second binding reagent can be the same or a different binding material as used in the aforesaid reagent except that when the same, it is, of course, ultimately different as an assay reagent by being labeled with the second label. When employing an antibody reagent selective for binding intercalation complexes for both binding reagents, it will normally be desired to employ a probe which has been modified to have the intercalator chemically linked to the probe, as opposed to being added as a soluble compound.

Alternatively, the second binding reagent can be any material which binds the probe. Thus, it can be an antibody reagent that binds to nucleic acids nonspecifically such as anti-DNA·DNA. Another embodiment will employ a probe comprising a specific binding ligand moiety with the nonspecific binding reagent then being a binding partner for such ligand moiety. In such case, the ligand moiety/binding partner pair can be selected from such known materials as haptens/antibodies, antigens/antibodies, biotin/avidin, lectins/polysaccharides, hormones/receptor proteins, and the like. In this respect, reference is made to British Pat. 2,019,408; European Patent Applns. 63,879 and 97,373 and PCT Published Applns. 83-1459 and 83-2286.

While it is generally preferred that the critical microenvironment of the bound hybrid be created in a portion or the entirety of the hybridized complementary region of the sample/probe duplex, in principle such microenvironment can also occur at the boundary of such complementary region and the flanking region. This can occur employing binding reagents as discussed above. Additionally, one can use as the nonspecific binding reagent a binding substance for a binding site on double stranded flanking regions of the probe. Examples of such binding site/binding partner pairs are promotor sequences (e.g., lac-promotor, trp-promoters, and promoters associated with bacteriophages)/polymerase; lac operator/lac repressor protein; and antigenic nucleotides and sequences (e.g., Z-DNA and rare nucleotides such as 5-halodeoxyuridines)/antibodies thereto.

With reference to the drawings and the examples which follow, a few specific embodiments of the present assay scheme can be described.

MS-1353

The method depicted in Fig. 1 employs an unmodified polynucleotide probe which is either RNA or DNA when the sample sequence of interest is RNA or when the sample sequence is DNA the probe is RNA. The binding reagents are the same or different antibody reagents (Ab) selective for RNA·DNA or RNA·RNA hybrids as the case may be. One antibody reagent is labeled with glucose oxidase (GOD) and the other with peroxidase (POD). In the presence of glucose and a color indicator for hydrogen peroxide, the rate of color development will be dependent upon the extent of hybridization that takes place. In the bound hybrid, glucose oxidase and peroxidase are brought within a distance $a$ which results in an increased rate of color development due to rapid diffusion of hydrogen peroxide to peroxidase. In the bulk medium, hydrogen peroxide must on average diffuse distance $b$ in order to cause a color development event which reflects in a significantly different rate. Optionally, catalase can be included in the bulk solution at concentrations sufficient to effectively destroy all hydrogen peroxide that diffuses away from the microenvironment of the bound hybrid to reduce background color formation.

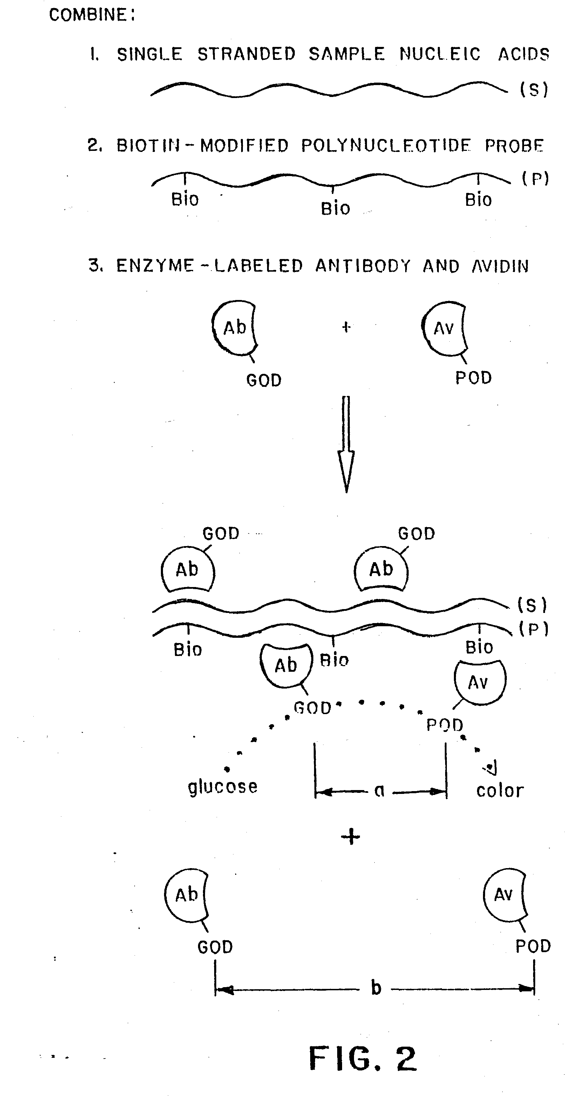

The use of a ligand-modified probe is illustrated in Fig. 2, with biotin (Bio) being the selected representative ligand. In this case the GOD-labeled binding reagent is again an antibody to RNA·DNA or RNA·RNA as the case may be, and the other binding reagent is POD-labeled avidin (Av). The color development readout will be understood as in the case of method A.

MS-1353

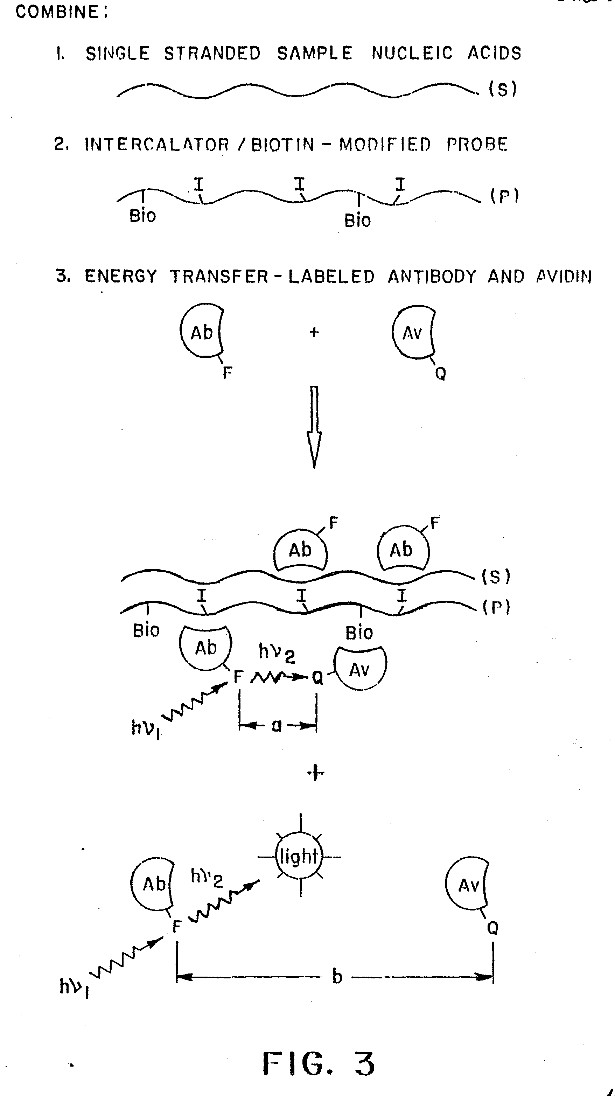

In the method shown in Fig. 3, the probe is doubly modified, with both biotin as in method B and a chemically linked intercalator (I). An antibody to intercalation complexes involving I is labeled with a fluorescer (F) and avidin is labeled with a quencher (Q). The fluorescer and quencher labels are brought into energy transfer distance $a$ in the bound hybrid such that upon irradiation with light of a first wavelength ($h\nu_1$), the emitted energy ($h\nu_2$) is absorbed by the quencher and not detected. On the other hand, fluorescer-labeled antibody in the bulk solution remains on average distance $b$ from the quencher, which is not close enough for efficient energy transfer, and the fluorescence emission is observed. The amount of $h\nu_2$ light detected is inversely related to the amount of hybridization that occurs.

*Reaction Mixture*

The test sample to be assayed can be any medium of interest, and will usually be a liquid sample of medical, veterinary, environmental, nutritional, or industrial significance. Human and animal specimens and body fluids particularly can be assayed by the present method, including urine, blood (serum or plasma), amniotic fluid, milk, cerebrospinal fluid, sputum, fecal matter, lung aspirates, throat swabs, genital swabs and exudates, rectal swabs, and nasopharnygal aspirates. Where the test sample obtained from the patient or other source to be tested contains principally double stranded nucleic acids, such as contained in cells, the sample will be treated to denature the nucleic acids, and if necessary first to release nucleic acids from cells. Denaturation of nucleic acids is preferably accomplished by heating in boiling water or alkali treatment (e.g., 0.1 N sodium hydroxide), which if desired, can simultaneously be used to lyse cells. Also, release of nucleic acids can, for example, be obtained by mechanical disruption (freeze/thaw, abrasion, sonication), physical/chemical disruption (detergents such as Triton, Tween, sodium dodecylsulfate, alkali treatment, osmotic shock, or heat), or enzymatic lysis (lysozyme, proteinase K, pepsin). The resulting test medium will contain nucleic acids in single stranded form which can then be assayed according to the present hybridization method. In those situations where RNA·DNA hybrids are to be detected with labeled

MS-1353

antibody reagents, mRNA and rRNA in the sample can be removed from participating in the binding reactions by conventional methods such as treatment with alkaline conditions, e.g., the same conditions used to denature the nucleic acids in the sample.

As is known in the art, various hybridization conditions can be employed in the assay. Typically, hybridization will proceed at slightly elevated temperatures, e.g., between about 35 and 75°C and usually around 65°C, in a solution comprising buffer at pH between about 6 and 8 and with appropriate ionic strength (e.g., 5XSSC where 1XSSC = 0.15M sodium chloride and 0.015M sodium citrate, pH 7.0). In cases where lower hybridization temperatures are desirable, hydrogen bonding reagents such as dimethyl sulfoxide and formamide can be included. The degree of complementarity between the sample and probe strands required for hybridization to occur depends on the stringency of the conditions. Factors which determine stringency are known in the art.

Normally, the temperature conditions selected for hybridization will be incompatible with the binding of the anti-hybrid reagent to formed hybrids and detection of the label response. Accordingly, the anti-hybrid binding step and label detection step will proceed after completion of the hybridization step. The reaction mixture will usually be brought to a temperature in the range of from about 3°C to about 40°C and the binding and detection steps then performed. Dilution of the hybridization mixture prior to addition of the antibody reagent is desirable when the salt and/or formamide concentrations are high enough to

MS-1353

interfere significantly with the antibody binding
reaction.

*Reagent System*

The present invention additionally provides a
reagent system, i.e., reagent combination or means,
comprising all of the essential elements required
to conduct a desired assay method. The reagent
system is presented in a commercially packaged
form, as a composition or admixture where the
compatability of the reagents will allow, in a test
device configuration, or more usually as a test
kit, i.e., a packaged combination of one or more
containers, devices, or the like holding the
necessary reagents, and usually including written
instructions for the performance of assays.
Reagent systems of the present invention include
all configurations and compositions for performing
the various hybridization formats described herein.

In all cases, the reagent system will comprise
(1) a nucleic acid probe as described herein, and
(2) the first and second labeled binding reagents.
A test kit form of the system can additionally
include ancillary chemicals such as the components
of the hybridization solution and denaturation
agents capable of converting double stranded
nucleic acids in a test sample into single stranded
form. Preferably, there is included a chemical
lysing and denaturing agent, e.g., alkali, for
treating the sample to release single stranded
nucleic acid therefrom.

MS-1353

The present invention will now be illustrated, but is not intended to be limited, by the following examples.

*Example I*

Hybridization Assay for Bacterial RNA Monitored with Labeled Antibodies to RNA·DNA Hybrid

A. Monoclonal antibody to RNA·DNA hybrid is raised by the method of Stuart et al (1981) PNAS 78, 3751. Antibody is isolated from ascites fluid by affinity chromatography on protein A Sepharose available from Pharmacia Fine Chemicals, Piscataway, NJ. About 1 milliliter (ml) of ascites fluid per ml of protein A Sepharose is applied to the column and then the column is washed with 50 millimolar (mM) Bicine buffer, pH 8.0, 0.15 molar (M) NaCl until the absorbance of the effluent at 280 nanometers (nm) is less than 0.05. The bound antibody is eluted with 0.1M glycine buffer, pH 3.0 and antibody is detected by measurement of absorbance at 280 nm. The antibody pool is adjusted to pH 5.0 or above by addition of 1M Bicine buffer, pH 8.5. The pool is dialyzed into 0.1M borate buffer, pH 9.0

The antibody is labeled lightly with 5-(4,6-dichlorotriazin-2-yl)-aminofluorescein (DTAF) (Molecular Probes, Inc, Junction City, Oregon) by the method of Blakeslee and Baines (1976) J. Immunol. Meth. 13, 305. A solution containing 1.44 micromoles (μmoles) of DTAF in 361 microliters (μL) of borate buffer is added to 2.4

MS-1353

ml of 0.014 mM antibody and allowed to react for one hour at 25°C. The reaction mixture is chromatographed on a 1 x 25 cm Biogel P-6DG (BioRad Laboratories, Richmond, CA) column with 0.1M sodium phosphate buffer, pH 7.0, 0.1M NaCl as the eluent. Absorbances at 280 nm of 1 ml fractions are monitored and those comprising the first peak are pooled. The molar DTAF/protein ratio is determined by the method of The and Feltkamp (1970) Immunology 18, 865.

Free thiol groups are introduced into the antibody by reaction with N-succinimidyl 3-(2-pyridyldithio)propionate and subsequent mild reduction with dithiothreitol [Carlson et al (1978) Biochem. J. 173, 723].

B.   A glucose oxidase antibody conjugate is prepared as follows. Maleimide groups are introduced onto glucose oxidase (available from Miles Scientific, Naperville, IL) by reaction with the N-hydroxysuccinimide ester of N-(4-carboxycyclohexylmethyl)-maleimide (SMCC) as described by [Yoshitoake, et al, (1979) Eur. J. Biochem. 101, 395 (1979)]. This glucose oxidase is immediately combined with a two-fold molar excess of the thiol derivatized antibody described above. The reaction conditions and purification methods of Yoshitake et al are followed. Enzyme content of the purified conjugate is determined by measurement of enzyme activity and the antibody content is determined by measurement of the DTAF content as described by The and Feltkamp, *supra*.

MS-1353

C.  Horse radish peroxidase is reacted with SMCC and coupled to the thiol derivatized antibody by the method of Ishikawa et al (1983) J. Immunoassay 4:209.

D.  A 565 base pair fragment from a gene for the 16s ribosomal RNA from E. coli has been cloned between Hind III sites of a pBR322 vector [Brosius (1978) Proc. Nat'l. Acad. Sci. 75:4801]. The fragment is excised by digestion with Hind III restriction endonuclease.

E.  A urine specimen containing greater than $10^5$ bacteria per ml is diluted three-fold into an enriched culture broth and incubated at 35°C until the cell population reached about $10^9$ organisms per ml. Aliquots of the culture are transferred to a series of tubes to give $10^5$ to $10^9$ bacteria per tube. The tubes are centrifiged at 10,000 x g for 10 minutes and the supernatants are discarded. The cells are lysed in 20 μl per tube of 10 milligrams (mg) egg white lysozyme (Sigma Chemical Co., St. Louis, MO) in 10 mM Tris-hydrochloride buffer, pH 8.0, 0.1M NaCl and 5 mM ethylenediaminetetraacetic acid. Fifteen minutes later, 80 μl of a solution composed of 60% formamide and 40% 0.16 M sodium phosphate buffer, pH 6.5, 1.44 M NaCl, and 0.1% (w/v) sodium dodecylsulfate.

The DNA probe described in section D above, in 0.2M sodium phosphate buffer, pH 6.5, is denatured in a boiling water bath for 5 minutes and cooled on ice. Twenty microliter aliquots (80 ng DNA) are added to each tube and incubated at 55°C for 18 hours. Then 500 μl of  peroxidase labeled antibody

MS-1353

and glucose oxidase labeled antibody are added to each tube and allowed to stand at room temperature for 2 hours. The concentrations of these enzyme conjugates are optimized in preliminary experiments to give optimum signal to background ratios.

The following substrate reagent is prepared and used within 3 hours: 0.1M sodium phosphate, pH 6.5, 0.1% bovine serum albumin, 20 mM 3,5-dichloro-2-hydroxybenzene sulfonate, 0.1M glucose, 0.01 unit catalase/ml and 0.2 mM 4-aminoantipyrine. One milliliter of substrate reagent is added to each tube and incubated at 37°C for 30 minutes. The the absorbances at 510 nm are recorded. As the number of bacteria per tube increases the amount of ribosomal RNA·DNA hybrid will increase and as a result the absorbance will be increased.

*Example II*

Hybridization Assay for Detection of Bacterial Ribosomal RNA Using a Biotinylated
DNA Probe

A.    Biotin-11-dUTP and streptavidin-horse radish peroxidase conjugate are available from Enzo Biochem., Inc., New York, NY.

B.    The 565 base pair probe for ribosomal RNA is labeled with biotin-11-dUTP by the method of Langer et al (1981) Proc. Nat'l. Acad. Sci., 78:6633.

C.    Bacteria are processed as outlined in Example I, section E above.  The lysates are combined with 80 μl of a solution composed of 60% formamide and 40% 0.16 M sodium phosphate buffer, pH 6.5, 1.44 M NaCl and 0.1% (w/v) sodium dodecylsulfate.  The biotinylated DNA probe is denatured in a boiling water bath for 5 minutes and then cooled on ice.  The probe is in 0.2M sodium phosphate buffer, pH 6.5 and 20 μl aliquots containing 80 ng probe are added to each extract and incubated at 65°C for 18 hours.  Then 500 μl of peroxidase labeled streptavidin and glucose oxidase labeled antibody against RNA·DNA hybrid is added to each tube and allowed to stand at room temperature for 2 hours.  The concentrations of these enzyme conjugates are optimized in preliminary experiments to give maximum signal to background ratios.

MS-1353

The enzyme reactions are initiated by addition of 1 ml of the substrate reagent described in Example I, section D. These reactions are allowed to proceed at 37°C for 30 minutes and then the absorbances at 510 nm are recorded. The absorbances will increase as the number of bacteria added per tube increases.

*Example III*

Hybridization Assay for Bacterial Ribosomal RNA Using a Probe Intercalated with Ethidium Bromide

A. Calf thymus DNA (Sigma Chemical Co.) is treated with pronase to digest protein contaminants and then precipitated in ethanol. The DNA is dissolved in 20 mM tris-hydrochloride buffer, pH 8.0, 0.2M NaCl.

A photoreactive derivative of ethidium bromide, 8-azidoethidium, is prepared by the method of Graves et al (1977) Biochim. Biophys. Acta, 479:98. A solution is prepared containing about 1 mM base pairs of the DNA and 0.5 mM 8-azidoethidium. This solution is stirred in a glass reaction vessel immersed in a water bath maintained at 20-30°C and is photolyzed 10 to 20 cm from a 150 watt spotlight for 60 minutes. Following the photolysis the noncovalently bound ethidium derivatives are removed by extractions with water saturated $n$-butanol. The amount of ethidium coupled to the DNA is estimated using the extinction coefficients of $E_{490} = 4 \times 10^3 M^{-1} cm^{-1}$ for photolyzed ethidium azide, the relationship

between $A_{260}$ and $A_{490}$ for photolyzed ethidium bound to DNA $[A_{260} = (A_{490} \times 3.4)-0.011]$, and $E_{260} = 1.32 \times 10^4$ $M^{-1}$ $cm^{-1}$ for the concentration of DNA base pairs of a given DNA being labeled.

B.   Preparation of methylated thyroglobulin One hundred milligrams of bovine thyroglobulin (Sigma) is combined with 10 ml of anhydrous methanol and 400 µl of 2.55 M HCl in methanol. This mixture is stirred on a rotary mixer at room temperature for 5 days.  The precipitate is collected by centrifugation and washed twice with methanol and twice with ethanol.  Then it is dried under vacuum overnight.  About 82 mg of dry powder is obtained.

C.   An immunogen is prepared by combining the DNA-ethidium complex with methylated thyroglobulin as outlined by Stöller (1980) Methods in Enzymology 70:70.  Twenty to fifty micrograms of the immunogen in Freunds adjuvant are injected per mouse and booster injections are given weekly beginning two weeks after the original immunization.  Antibody titers are measured by an ELISA procedure and hybridomas are produced and screened for antibody by standard procedures [Galfre and Milstein, (1981) Meth. Enzymol., 73:1; Poirier, et al (1982) Proc. Nat'l. Acad. Sci., 79:6443].  Selection procedures were designed for antibodies which bind the DNA-ethidium complex with association constants of $10^{10}$ or greater but do not bind to single or double stranded DNA or the ethidium residue alone.

Antibody is isolated from ascites fluid as outlined in Example I, section A for antibody to

MS-1353

RNA·DNA hybrid. This antibody is also labeled lightly with DTAF and coupled to glucose oxidase.

D.   The 565 base pair fragment from the pKK115 plasmid is cloned into the Hind III site of M13 mp9 [Messing and Vieria, (1982) Gene 19, 269; commercially available from New England Biolabs, Beverly, MA]. The fragment inserts in two orientations and a clone with the virion DNA sequence complementary to ribosomal RNA is selected for further work.

The modified M13 mp9 is propagated in E. coli JM103 [Δlac, pro)thi, strA, supE, endA, sbcB15, hsdR4, F'traD36, proAB, lacIq, ZΔM15] available from Bethesda Research Laboratories, Gaithersburg, MD. The phage is isolated from culture broth by precipitation with polyethylene glycol and the virion DNA is purified by phenol/chloroform extraction [Maniatis et al (1982) Molecular Cloning, A Laboratory Manual, Cold Spring Harbor].

An oligonucleotide primer (CACAATTCCACACAAC; available from New England Biolabs) is complementary to the M13 mp9 vector on the 5'-OH side of the inserted ribosomal RNA probe. This primer is used to initiate replication of the M 13 sequence by E. coli DNA polymerase (Klenow fragment) in the presence of limiting quantities of deoxynucleotides triphosphates. The replication is limited to an extent that the inserted ribosomal RNA probe remains single stranded and available for hybridization [Hu and Messing (1982) Gene 17:271].

This modified M 13 probe is mixed with 8-azidoethidium, photolyzed and purified as outlined in section A above.

MS-1353

E. Bacteria are prepared and lysed as described in Example I, part A. The lysates are mixed with 80 µl of the formamide hybridization solution described in Example I, Part E. Twenty microliters of the ethidium-probe complex (100 ng DNA) (section D) is added to the extracts and incubated at 65°C for 18 hours and then 500 µl of peroxidase labeled antibody to RNA·DNA and glucose oxidase labeled antibody to ethidium-DNA complex is added to each tube. The mixtures are allowed to stand at room temperature for 2 hours.

At the end of this incubation, 1.0 ml of the substrate reagent, Example I, part E, is added and incubated at 37°C for 30 minutes. Then the absorbance at 510 nm is recorded. As the number of bacteria per tube is increased, the absorbance will increase.

*Example IV*

Hybridization Assays for Cylomegalovirus Monitored with a Fluorescer-Quencher Pair

A. A 1500 base pair fragment of the cytomegalovirus genome is cloned into the M13mp8 vector. A complementary copy of the inserted fragment is synthesized *in vitro* with DNA polymerase in the presence of a biotinylated nucleotide triphosphate to provide a biotinylated probe. This probe is modified further by covalent incorporation of ethidium residues as intercalation complexes.

An EcoRI digest of cytomegalovirus DNA is prepared and the fragments are cloned into the

MS-1353

plasmid pACYC184 [Tamashiro et al (1982) J. Virology 42:547]. The plasmids are propogated in E. coli strain HB101 Rec A⁻ and the EcoRI e fragment described in the Tamashiro reference is used for preparation of the probe. The fragment is removed from the plasmid by digestion with EcoRI restriction enzyme and cloned into the EcoRI site of the M13mp8 vector (New England Biolabs) which is propagated in the E. coli K12JM101 host. The virus is isolated from the culture fluid by precipitation with polyethyleneglycol and the DNA is purified by phenol/chloroform extraction.

The DNA is annealed with a 17 base primer with the sequence GTAAAACGACGGCCAGT (New England Biolabs) which is complementary to a region of the M13mp8 sequence near the 3'-OH end of the EcoRI e insert. The DNA is 20 mM Tris-hydrochloride buffer, pH 8.0, containing 10 mM MgCl$_2$ is combined with a molar excess of the primer and incubated at 55°C for 45 minutes [Bankier and Barrell (1983) Techniques in Nucleic Acid Biochemistry, Elsevier, Ireland]. Then the reaction mixture is made 15 mM in dATP, dCTP, dGTP and Bio-11-dUTP (available from Enzo Biochem) and finally the Klenow fragment of DNA polymerase I is added. This reaction is incubated at 25°C for a period determined in preliminary experiments. In these experiments samples are taken from the reaction mixture at various times and electrophoresed in alkaline agarose gel [Maniatis et al, supra]. The objective is to find reaction conditions which give biotin labeled probes that extend through the EcoRI e insert. This procedure will provide biotin labeled DNA with some variation in length; however,

MS-1353

extension of the labeled DNA beyond the EcoRI e insert into the M13 sequence is acceptable for the present purpose.

The next step is the covalent coupling of ethidium residues to the biotin labeled DNA as intercalation complexes. This is accomplished by photolysis of the DNA with 8-azidoethidium prepared by the method of Graves et al, supra. The biotin labeled DNA is extracted with phenol/chloroform and precipitated with ethanol. It is dissolved in 20 mM Tris-hydrochloride buffer, pH 8.0, 0.2 M NaCl, to give about 50 µg DNA/ml and made 0.5 mM with 8-azidoethidium. The mixture is photolyzed for 1 hour 10 to 20 cm from a 150 watt spotlight. The mixture is stirred during this period in a glass reaction vessel immersed in a glass water bath. The bath maintains a reaction temperature of 20 to 30°C.

Then the noncovalently bound 8-azidoethidium and photolysis by-products are removed by the successive extractions with water saturated n-butanol. The DNA is precipitated with ethanol and dissolved in the Tris-buffer. The covalently bound ethidium is measured spectrophotometrically as described in Example III, section A.

8-Azidoethidium binds covalently to the DNA almost exclusively in the double stranded region where intercalation complexes can form. The objective is to covalently couple one ethidium residue per 10 to 50 base pairs. Since the photolytic reaction goes nearly to completion in one hour, the incorporation can be decreased by reducing the reaction time or by reducing the 8-azidoethidium concentration. The incorporation can be increased by repeating the photolysis with fresh 8-azidoethidium.

The final step is separation of the biotin labeled/ethidium modified probe from the M13mp8 template. The labeled probe is substantially smaller than the M13mp8 template and can be isolated by alkaline agarose gel electrophoresis [Maniatis et al, supra]. The gel containing the probe is cut from the agarose gel slab and recovered by electroelution as described in the Maniatis reference.

B.    Fluorescein labeled antibody to ethidium-DNA intercalation complex.

Monoclonal antibody to ethidium-DNA complex described in Example III, part B above is dialyzed into 0.1 M sodium borate buffer, pH 9.0, and 0.5 ml containing 5 mg antibody is combined with 0.5 ml of 10 mM 5(4,6-dichlorotriazin-2-yl)-aminofluorescein (Molecular Probes, Inc., Junction City, OR) in the borate buffer. The mixture is allowed to stand for 7 hours at room temperature and then it is chromotographed on a 1 x 26 cm column of Biogel P6DG resin (Bio-Rad Laboratories) in 0.1 M Tris-hydrochloride buffer, pH 8.0. The absorbances at 280 nm of 1.0 mL fractions are measured and fractions from the first eluted peak are pooled. The fluorescein/protein ratio is measured spectrophotometrically by the method of The and Feltkamp (1970) Immunology 18:865.

C.   Preparation of streptavidin labeled with
     4,5-dimethoxy-6-carboxyfluorescein.


The N-hydroxysuccinimide ester of
4',5'-dimethoxy-6-carboxyfluorescein is synthesized
by the method of Khanna and Ullman (1980) Anal.
Biochem. 108:156.  (The synthesis actually gives a
mixture of the 5 and 6- carboxyfluorescein
isomers).  Streptavidin isolated from Streptomyces
avidini [Hoffman et al (1980) Proc. Nat'l. Acad.
Sci. 77:4666] is labeled with the
N-hydroxysuccinimide ester of
4',5'-dimethoxy-6-carboxyfluorescein by the method
described by Khanna and Ullman for labeling
immunoglobulins.


D.   Hybridization assay for cytomegalovirus
     in urine.


The procedure uses centrifugation to isolate
the virus from urine and the viral DNA is
hybridized in solution with the biotin
labeled/ethidium modified probe.  Then the labeled
avidin and antibody to ethidium-DNA intercalation
complex are allowed to bind to the hybrids formed
and fluorescence is used to estimate the amount of
hybrid present.
     Cellular and particulate materials are removed
from urine by centrifugation in a Sorvall GLC-3
centrifuge at 3000 rpm for five minutes.  The
supernatant is placed in a polyallomer
ultracentrifuge tube and run at 25,000 rpm in a
Beckman Ti50 rotor for 75 minutes.  The pellets are

MS-1353

dissolved in 0.1 M NaOH and incubated at 37°C for 30 minutes.

One hundred fifty microliters of 0.2 M sodium phosphate buffer, pH 6.0, containing 1.8 M NaCl, 0.1% sodium dodecylsulfate (w/v) and 1 mM EDTA is added. Then 20 µL of the biotin labeled/ethidium modified probe (50 mg) is added and the hybridization mixture is incubated at 65°C for 10 hours. After the hybridization reaction, 650 µL of 0.1 M Tris-hydrochloride buffer, pH 8.2, containing the labeled avidin and antibody to ethidium-DNA intercalation complex is added. The concentrations of the labeled proteins in this reagent are optimized in preliminary experiments to maximize the fluorescence quenching to background ratio. The protein binding reactions are allowed to proceed at room temperature for 1 hour.

Then the fluorescence of the reaction mixture is recorded using 495 nm light for excitation and 519 nm for emission. A reference assay is run in parallel with a urine known to be free of cytomegalovirus and the fluorescence signal obtained with this reference assay will be higher than that for samples containing the virus, thus the fluorescence signal will increase as the virus level decreases.

The present invention has been particularly described and exemplified above. Obviously many other variations and modifications of the invention may be made without departing from the spirit and scope thereof.

MS-1353

PROPOSED CLAIMS FOR EPO, DENMARK,
FINLAND, IRELAND, AND NORWAY

1. A method for detecting a particular polynucleotide sequence in a test sample comprising single stranded nucleic acids, comprising the steps of contacting the test sample with a nucleic acid probe comprising at least one single stranded base sequence that is substantially complementary to the sequence to be detected, and determining the resulting hybrids,

characterized in that the hybrids are formed to have binding sites for two specific binding reagents, any hybrid that is formed is contacted with the two specific binding reagents, one of which comprises a first label and the other comprising a second label, interaction between the first and second labels providing a detectable response which is measurably different when the two labeled reagents are both bound to the same hybrid, compared to when the two labeled reagents are not so bound, and the detectable response is measured as a function of the presence of the sequence to be detected in the sample.

2. The method of Claim 1 wherein the first label participates in a first chemical reaction that produces a diffusible product which is a participant in a second chemical reaction with the second label to produce a product that provides the detectable response, the first and second labels preferably being catalysts for the first and second chemical reactions, respectively.

3. The method of Claim 1 wherein the first and second labels participate in an energy transfer interaction.

4. The method of Claim 1 wherein one of the specific binding reagents is an antibody reagent.

MS-1353

5.    The method of Claim 4 wherein the antibody reagent is one that is selective for binding DNA·RNA hybrids wherein one of the probe and the sequence to be detected is DNA and the other is RNA, or is one that is selective for binding RNA·RNA hybrids wherein both the probe and the sequence to be detected are RNA.

6.    The method of Claim 4 wherein the antibody reagent is one that is selective for binding intercalation complexes wherein the duplexes formed in the assay comprise a nucleic acid intercalator bound thereto in the form of intercalation complexes, preferably the intercalator being chemically linked to the probe in its single stranded complementary region whereby upon hybridization with the sequence to be detected said intercalation complexes are formed in the resulting hybrid.

7.    The method of either of Claim 5 or 6 wherein (i) the other specific binding reagent comprises the same antibody reagent, (ii) the nucleic acid probe comprises a specific binding ligand moiety and the other specific binding reagent is a binding partner for such ligand moiety, or (iii) the nucleic acid probe comprises a double stranded portion having a binding site for a protein and the other specific binding reagent is such protein.

8.    The method of Claim 1 wherein the test sample comprises a biological sample which has been subject to conditions to release and denature nucleic acids present therein.

9.    A reagent system for detecting a particular polynucleotide sequence in a test sample, comprising a nucleic acid probe comprising at least one single stranded base sequence that is substantially complementary to the sequences to be detected,

characterized by comprising additionally first and second specific binding reagents capable of binding to hybrids formed between the sequence to be detected in the sample and the probe, such binding reagents comprising first and second labels, respectively, which interact with each other to provide a detectable response which is measurably different when the two labeled reagents are both bound to the same hybrid compared to when the two labeled reagents are not so bound.

10. The reagent system of Claim 9 wherein the first label participates in a first chemical reaction that produces a diffusible product which is a participant in a second chemical reaction with the second label to produce a product that provides the detectable response, the first and second labels preferably being catalysts for the first and second chemical reactions, respectively.

11. The reagent system of Claim 9 wherein the first and second labels participate in an energy transfer interaction.

12. The reagent system of Claim 9 wherein one of the specific binding reagents is an antibody reagent.

13. The reagent system of Claim 12 wherein the antibody reagent is one that is selective for binding DNA·RNA hybrids wherein one of the probe and the sequence to be detected is DNA and the other is RNA, or is one that is selective for binding RNA·RNA hybrids wherein both the probe and the sequence to be detected are RNA.

14. The reagent system of Claim 12 wherein the antibody reagent is one that is selective for binding intercalation complexes wherein the duplexes formed in the assay comprise a nucleic acid intercalator bound thereto in the form of intercalation complexes, preferably the intercalator being chemically linked to the probe in its single stranded complementary region whereby upon hybridization with the sequence to be detected said intercalation complexes are formed in the resulting hybrid.

15. The reagent system of either of Claims 13 or 14 wherein (i) the other specific binding reagent comprises the same antibody reagent, (ii) the nucleic acid probe comprises a specific binding ligand moiety and the other specific binding reagent is a binding partner for such ligand moiety, or (iii) the nucleic acid probe comprises a double stranded portion having a binding site for a protein and the other specific binding reagent is such protein.

/mc

MS-1353

COMBINE:

1. SINGLE STRANDED SAMPLE NUCLEIC ACIDS

(S)

2. POLYNUCLEOTIDE PROBE

(P)

3. ENZYME LABELED ANTIBODIES

FIG. 1

COMBINE:

I. SINGLE STRANDED SAMPLE NUCLEIC ACIDS

〜〜〜〜〜〜〜 (S)

2. BIOTIN-MODIFIED POLYNUCLEOTIDE PROBE

〜〜〜〜〜〜〜 (P)
Bio          Bio          Bio

3. ENZYME-LABELED ANTIBODY AND AVIDIN

Ab          +          Av
GOD                    POD

GOD               GOD
Ab                Ab
〜〜〜〜〜〜〜 (S)
〜〜〜〜〜〜〜 (P)
Bio        Ab  Bio              Bio
GOD                    Av
POD

glucose    ←— a —→    color

+

Ab                              Av
GOD                            POD
←——————— b ———————→

FIG. 2

FIG. 3